# EUROPÄISCHE PATENTANMELDUNG

(11) **EP 1 712 543 A1**
(43) Veröffentlichungstag der Anmeldung: **18.10.2006**
(21) Anmeldenummer: 06005454.1
(22) Anmeldetag: 17.03.2006
(51) Int. Cl.: C07C 231/02, C07C 233/20, C08G 65/333

(54) **Verfahren zur Herstellung von Amiden basierend auf Polytheraminen und (Meth)acrylsäure**

(30) Priorität: 15.04.2005 DE 102005017453
(71) Anmelder: Clariant Produkte (Deutschland) GmbH, 65929 Frankfurt am Main (DE)
(72) Erfinder: Falk, Uwe, 63486 Bruchköbel (DE); Glos, Martin, 45145 Essen (DE); Morschhäuser, Roman, 55122 Mainz (DE); Ritter, Helmut, 42111 Wuppertal (DE); Schmitz, Sarah, 40479 Düsseldorf (DE)
(74) Vertreter: Mikulecky, Klaus

(57) **Zusammenfassung**

Gegenstand der Erfindung ist ein Verfahren zur Herstellung von Amiden aus Polyetheraminen und (Meth)acrylsäure bzw. den entsprechenden Anhydriden, wobei als Energiequelle Mikrowellen verwendet werden.

## Beschreibung

Die vorliegende Erfindung betrifft ein Verfahren zur Herstellung von Amiden der Acrylsäure oder Methacrylsäure mit Polyetheraminen unter Verwendung von Mikrowellen als Energiequelle. Die so erhaltenen Amide dienen als Makromonomere, deren Eigenschaften durch die Wahl des geeigneten Polyetheramins variiert werden können.

Polyetheramine enthalten eine Polyalkylenglykolgruppe. Je nachdem aus welchen Bausteinen man Polyalkylenglykole aufbaut, kann man verschiedene Eigenschaften erhalten. So erhält man bei hohem Anteil an Polyethylenglykol wasserlösliche und bei Verwendung von Polypropylenglykolen wasserunlösliche Verbindungen. Außerdem lassen sich durch die Veränderung der Molmassen der Polyalkylenglykole ihr Schmelzpunkt und ihre Viskosität beeinflussen. Durch die Verwendung von verschiedenen Startalkoholen für die Alkoxylierung lassen sich tensidische Eigenschaften erzielen. Außerdem kann man mit mehrwertigen Alkoholen verzweigte Systeme aufbauen, die dann nach der Aminolyse und Amidierung zu Monomeren mit vernetzender Wirkung führen. Damit ergeben sich vielfältige Möglichkeiten, durch Makromonomere auf Basis von Polyalkylenglykolen die Eigenschaften eines darauf basierenden Polymers gezielt zu beeinflussen.

Unter den Amiden der olefinisch ungesättigten Carbonsäuren sind besonders die (Meth)acrylsäureamide technisch interessant, da sie Ausgangsverbindungen zur Herstellung von Polymeren und Copolymere darstellen, die in vielfältigen Bereichen Anwendungen finden. Der Begriff (Meth)acrylsäureamide steht für Methacrylsäureamide und Acrylsäureamide.

Ein Vorteil der Amide gegenüber den entsprechenden Estern ist die größere Stabilität, die sich z.B. durch geringere Hydrolyseempfindlichkeit äußert.

Bei der Herstellung der Amide ausgehend von Aminen mit (Meth)acrylsäure sind verschiedene Probleme zu beobachten. Eine unerwünschte Nebenreaktion ist die Michael-Addition der Amine an die Doppelbindung der ungesättigten Carbonsäure. Außerdem neigen (Meth)acrylsäurederivate unter dem Einfluss von Hitze oder Licht zur Polymerisation. Vor allem bei der Herstellung und destillativen Reinigung sind sie Temperaturen ausgesetzt, die leicht eine unerwünschte Polymerisation auslösen können.

Der Stand der Technik offenbart verschiedene Verfahren zur Herstellung von (Meth)Acrylsäureamiden.

DE-A-28 16 516 beschreibt ein Verfahren, bei dem (Meth)acrylsäureamide ausgehend von Alkyl(meth)acrylsäureestern hergestellt werden. Hierbei wird zu Vermeidung von Michael-Addukten in Gegenwart von Dibutylzinnoxid gearbeitet.

DE-A-31 23 970 beschreibt ein Verfahren, bei dem (Meth)acrylsäureamide ausgehend von Alkyl(meth)acrylsäureestern hergestellt werden. Hierbei wird zu Vermeidung von Michael-Addukten in Gegenwart von Verbindungen der Metalle der 4. Nebengruppe und/oder Verbindungen der Metalle Blei, Tantal und/oder Zink als Katalysatoren gearbeitet.

EP 0 992 480 A1 beschreibt ein Verfahren zur Herstellung von Estern der (Meth)acrylsäure unter Verwendung von Mikrowellen als Energiequelle. Es werden dort aber keine Amide nach dieser Methode hergestellt.

Von besonderem Interesse sind Makromonomere auf Basis von Polyalkylenglykolen, da sich hier durch Verwendung von hydrophoben und/oder hydrophilen Alkylenoxiden oder Ausgangsverbindungen mit den entsprechenden Eigenschaften bei der Herstellung des zugrunde liegenden Polyalkylenglykols die Hydrophilie/Hydrophobie des Makromonomers gezielt beeinflussen lässt. Bei der Verwendung von Polyalkylenglykolen in chemischen Umsetzungen besteht oftmals das Problem der erhöhten Polymerisationsneigung. Wegen der häufig hohen Molmassen sind die Substanzen relativ reaktionsträge und die Produkte lassen nicht destillativ reinigen. Daher werden an Synthesen mit Polyalkylenglykolen besondere Anforderungen gestellt.

Überraschenderweise wurde nun gefunden, dass Amide aus Polyetheraminen und (Meth)acrylsäure hergestellt werden können, indem man Mikrowellen als Energiequelle verwendet. Hierbei wird die Bildung von Michael-Additionsprodukten unterdrückt, so dass man die gewünschten Amide in hoher Ausbeute und Reinheit erhält.

Gegenstand der Erfindung ist somit ein Verfahren zur Herstellung von Amiden der (Meth)acrylsäure, indem man ein Polyetheramin der Formel 1 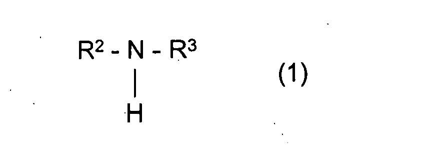 worin R² einen organischen Rest darstellt, der zwischen 2 und 600 Alkoxygruppen umfasst, und R³ Wasserstoff oder einen organischen Rest mit 1 bis 400 Kohlenstoffatomen darstellt, mit (Meth)Acrylsäure mischt, und das Gemisch mit Mikrowellen bestrahlt.

R² enthält 2 bis 600 Alkoxygruppen. Unter Alkoxygruppen wird vorliegend eine Einheit der Formel -(AO)- verstanden, worin A für eine C₂- bis C₄-Alkylengruppe steht. 2 bis 600 Alkoxygruppen bedeuten damit eine Struktureinheit der Formel - (AO)ₙ- mit n = 2 bis 600.

In der durch (A-O)ₙ wiedergegebenen Alkoxykette bedeutet A vorzugsweise einen Ethylen- oder Propylenrest, insbesondere einen Ethylenrest. Die Gesamtzahl von Alkoxyeinheiten liegt vorzugsweise zwischen 5 und 300, insbesondere zwischen 8 und 200. Bei der Alkoxykette kann es sich um eine Blockpolymerkette handeln, die alternierende Blöcke verschiedener Alkoxyeinheiten, vorzugsweise Ethoxy- und Propoxyeinheiten, aufweist. Es kann sich dabei auch um eine Kette mit statistischer Abfolge der Alkoxyeinheiten, oder ein Homopolymer handeln.

In einer bevorzugten Ausführungsform steht -(A-O)ₙ- für eine Alkoxykette der Formel 2

- (CH₂ - CH(CH₃) -O)ₐ - (CH₂- CH₂- O)_{b} - (CH₂ - CH(CH₃) - O)_{c} - (2)

worin
a eine Zahl von 0 bis 300, vorzugsweise 0 bis 80
b eine Zahl von 3 bis 300, vorzugsweise 3 bis 200
c eine Zahl von 0 bis 300, vorzugsweise 0 bis 80 bedeuten.

R³ ist Wasserstoff oder ein organischer Rest mit 1 bis 400 Kohlenstoffatomen. R³ kann neben Kohlenstoff und Wasserstoff auch Heteroatome wie Sauerstoff, Stickstoff, Phosphor oder Schwefel enthalten.
In einer bevorzugten Ausführungsform ist R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen.

In einer weiteren bevorzugten Ausführungsform entspricht R³ der gleichen- Definition wie R².

In einer weiteren bevorzugten Ausführungsform enthält R³ Aminogruppen. Vorzugsweise entspricht R³ dann der Formel 3 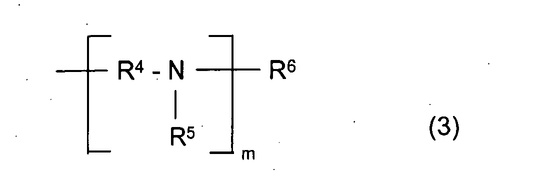 worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Alkoxygruppen umfassen kann und auch Heteroatome wie Sauerstoff, Stickstoff, Phosphor oder Schwefel enthalten können (im Grunde wie R³); und m für eine Zahl von 1 bis 10 steht.

Die im erfindungsgemäßen Verfahren aus einem Monoamin erhaltenen Produkte entsprechen der Formel 4 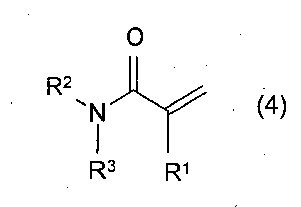 worin R² und R³ die oben angegebene Bedeutung haben und R¹ für Wasserstoff oder Methyl steht.

Ist das Polyetheramin ein Diamin der Formel 5 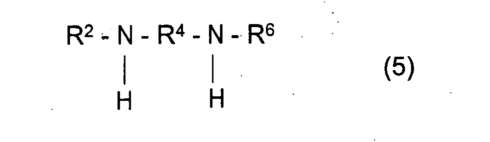 so entspricht das nach dem erfindungsgemäßen Verfahren erhaltene Produkt der Formel 6 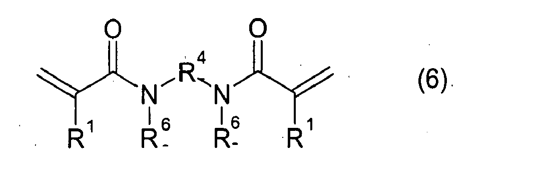 Entsprechend lassen sich aus tri-, tetra-, oder penta-funktionellen Aminen Tri-, Tetra-, oder Penta(meth)acrylamide herstellen. Damit ergibt sich beispielsweise eine Struktur der Formel 7 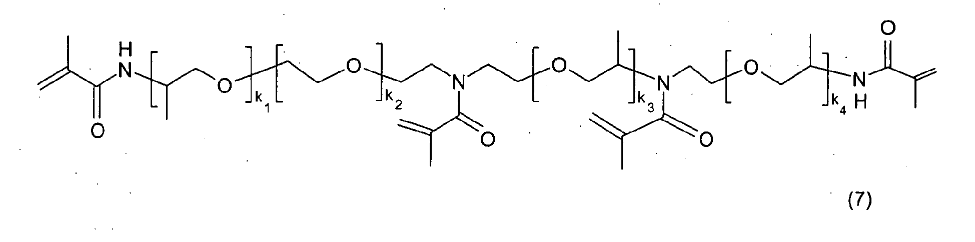 worin k₁, k₂, k₃ und k₄ ganze Zahlen darstellen, die in der Summe bis zu 600 ergeben.

In einer weiteren bevorzugten Ausführungsform steht das Amin der Formel (1) für ein Polyamin der Formel 8

R⁷(NHR⁸)ₙ (8)

worin R⁷ für einen n-wertigen organischen Rest mit 2 bis 400 Kohlenstoffatomen, der auch Heteroatome wie Sauerstoff, Stickstoff, Phosphor oder Schwefel enthalten kann, R⁸ für einen Rest wie R³, und n für eine ganze Zahl von 2 bis 20 steht.

Wenn R⁷ für alkoxyliertes Glycerin steht, so kann das Produkt des erfindungsgemäßen Verfahrens beispielsweise folgende Struktur aufweisen: 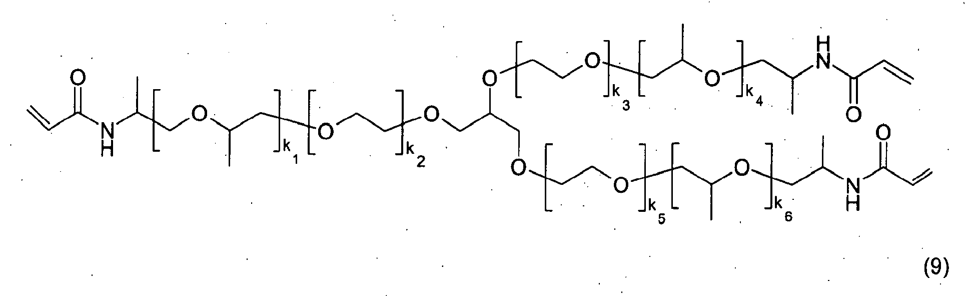 worin die Indices kₙ ganze zahlen sind, die in der Suinme bis zu 600 ergeben.

Als Polyetheramine können ein- oder mehrwertige Amine, die verzweigt, unverzweigt oder cyclisch, gesättigt oder ungesättigt sein können, eingesetzt werden. Solche Amine sind beispielsweise
- einwertige Amine, wie beispielsweise
   Alkylpolyalkylenglykolamine wie z.B. Methyltriethylenglykolamin, Bis(methyltriethylenglykol)amin, Butyltriethylenglykolamin, Laurylpolypropylenglykolamin, Methyltripropylenglykolamin, Phenlolpolypropylenglykolamin, iso-Tridecylpolypropylenglykolamin, Bis(methyltripropylenglykol)amin, N-Methyl-Methylpolypropylenglykolamin, . Methylpolypropylenglykolamin, Bis(methylpolypropylenglykol)amin, Methylpolyalkylenglykolamin mit statistischer oder blockartiger Verteilung der Ethylen- und Propylenglykol-Einheiten,
- zweiwertige Amine, wie beispielsweise
   Triethylenglykoldiamin, Tripropylenglykoldiamin, Polyethylenglykoldiamine; Polypropylenglykoldiamin, Polylalkylenglykoldiamin mit statistischer oder blockartiger Verteilung der Ethylen- und Propylenglykol-Einheiten, Butandiolpolyalkylenglykoldiamin, Resorcinpolyalkylenglykoldiamin,
- dreiwertige Amine, wie beispielsweise
   Glycerinpolyalkylenglykoltriamin mit statistischer oder blockartiger Verteilung der Ethylen- und Propylenglykol-Einheiten, Bis(triethylenglykolamin)amin, Bis(polyalkylenglykolamin)amin,
- vierwertige Amine, wie beispielsweise
   Pentaerythrolpolyalkylenglykol mit statistischer oder blockartiger Verteilung der Ethylen- und Propylenglykol-Einheiten, N,N'-Bis(polypropylenglykolamin)-polyalkylenglykoldiamin.

Das molare Verhältnis Amin : (Meth)Acrylsäure liegt vorzugsweise im Bereich 1: 0,2 bis 15, insbesondere im Bereich 1:0,8 bis 15.

Zur Vermeidung der Polymerisation während der Amidierungsreaktion können die nach dem Stand der Technik bekannten Stabilisatoren verwendet werden. Übliche Stabilisatoren sind N-Oxyle wie z.B. 4-Hydroxy-2,2,6,6-tetramethyl-1-oxyl-piperidin, 4-Oxo-2,2,6,6-tetramethylpiperidin-N-Oxy, Phenole und Naphthole wie z.B. Hydrochinon, Naphthochinon, p-Aminophenol, p-Nitrosophenol, 2-tert.-Butylphenol, 4-tert.-Butylphenol, 2,4-Di-tert.-Butylphenol, 2-Methyl-4-tert.-Buthylphenol, 4-Methyl-2,3-di-tert.-butylphenol, lonol K 65® , p-Methoxyphenol, Butylhydroxyanisol oder 4-Amine, wie z.B. N,N-Diphenylamin, Phenylendiamine, wie z.B. N,N'-Dialkyl-paraphenylendiamine, wobei die Alkylreste gleich oder verschieden sein können, Hydroxylamine, wie z.B. N,N-Diethylhydroxylamin, phosphorhaltige Verbindungen, wie z.B. Triphenylphosphin, Triphenylphosphit oder Triethylphosphit oder schwefelhaltige Verbindungen, wie z.B. Schwefeldioxid, Diphenylsulfid, Phenothiazin oder 5-tert.-Butyl-4-hydroxy-2-methylphenylsulfid sowie Irganox® -Typen, Cupferron-Typen® und Kupfer-Salze.

Diese Verbindungen können einzeln oder in Mischungen eingesetzt werden. Die Mengen reichen von 10 ppm bis 5 Gew.-% meistens von 50 ppm bis 3 Gew.-% bezogen auf das eingesetzte Amin. Die Reaktion kann in einer Inertgasatmosphäre (wie z.B. Stickstoff, Argon, Helium) oder auch gegebenenfalls unter Zugabe von Luft oder sauerstoffhaltigen Gasgemischen durchgeführt werden.

Die Amidierung kann ohne oder mit Katalysator durchgeführt werden. Hierbei können die nach dem Stand der Technik bekannten Katalysatoren verwendet werden. Übliche Katalysatoren sind Schwefelsäure, schweflige Säure, Dischwefelsäure, Polyschwefelsäuren, Schwefeltrioxid, Methansulfonsäure, Benzolsulfonsäure, C₁-C₃₀-Alkylbenzolsulfonsäure, Naphthalinsulfonsäure, Schwefelsäuremonoester von C₁-C₃₀-Alkoholen wie Dodecylsulfat, Phosphorsäure, phosphorige Säure, unterphosphorige Säure, Polyphosphorsäure, Phosphorsäureester von C₁-C₃₀-Alkoholen, Chlorwasserstoffsäure, Perchlorsäure, saure Ionentauscher, Heteropolysäuren, "solid super acids", sowie Salze dieser Säuren, Lewissäuren wie Bortrichlorid, Aluminiumsulfat und Eisentrichlorid.

Der Katalysator wird gegebenenfalls in Mengen von vorzugsweise 0,01 bis 10, insbesondere 0,05 bis 5 Gew.-% bezogen auf das gesamte Reaktionsgemisch eingesetzt.

Die Amidierungen können bei Temperaturen von 40 bis 250°C durchgeführt werden. Bevorzugt wird im Bereich von 80 bis 210°C gearbeitet. Die Amidierung wird vorzugsweise mit einem Überschuss an (Meth)acrylsäure durchgeführt. Vorzugsweise wird in einem Druckbereich von 1 mbar bis 10 bar gearbeitet.

Die Amidierung kann kontinuierlich oder diskontinuierlich durchgeführt werden.

Die Amidierung wird bevorzugt ohne Lösungsmittel durchgeführt. Es können aber auch Lösungsmittel verwendet werden.

Zur Erzeugung der Mikrowellen können die in der Technik üblichen Methoden und Apparate verwendet werden. Üblicherweise liegt die Frequenz der Mikrowellen im Bereich zwischen 300 MHz und 300 GHz, was einer Wellenlänge von 1 m bis 1 mm entspricht. Üblicherweise werden Frequenzen im Bereich von 850-950 bzw. 2300-2600 MHz verwendet, da diese Frequenzen nicht für Kommunikationszwecke beansprucht werden. Die Mikrowellen können nach den bekannten Methoden auf das Reaktionsmedium übertragen werden. So können sowohl multi-mode- als auch mono-mode-Geräte verwendet werden.

### Beispiele

Für die Beispiele 1 bis 5 wurde ein Mikrowellengenerator der Firma CEM, Modell "Discover" verwendet.
Für die Beispiele 6 bis 8 wurde ein Mikrowellengerät der Firma MLS, Modell "MLS-ETHOSplus" verwendet.

Für das Vergleichsbeispiel 1 wurde ein konventionelles Ölbad als Wärmequelle verwendet.

### Beispiel 1

1 g eines Methylpolyalkylenglykolamins (Ethylenglykol- Propylenglykol-Mischung) mit einer mittleren Molmasse von 750 g/mol und 0,34 g Methacrylsäure wurde in ein Druckgefäß mit Rührstab gefüllt. Das Druckgefäß wurde fest verschlossen und die Reaktionsmischung bei 290 W für 10 Minuten unter Rühren und Luftkühlung so bestrahlt, dass die maximale Temperatur ca. 210°C betrug. Durch Integration mittels ¹H-NMR-Spektroskopie konnte ein Umsetzungsgrad von > 98 % ermittelt werden.

### Beispiel 2

1 g eines Methylpolyalkylenglykolamins (Ethylenglykol- Propylenglykol-Mischung) mit einer mittleren Molmasse von 1250 g/mol und 0,4 g Methacrylsäure wurde in ein Druckgefäß mit Rührstab gefüllt. Das Druckgefäß wurde fest verschlossen und die Reaktionsmischung bei 290 W für 10 Minuten unter Rühren und Luftkühlung so bestrahlt, dass die maximale Temperatur ca. 170°C betrug. Durch Integration mittels ¹H-NMR-Spektroskopie konnte ein Umsetzungsgrad von > 98 % ermittelt werden.

### Beispiel 3

1 g eines Methylpolyalkylenglykolamins (Ethylenglykol- Propylenglykol-Mischung) mit einer mittleren Molmasse von 2000 g/mol und 0,2 g Methacrylsäure wurde in ein Druckgefäß mit Rührstab gefüllt. Das Druckgefäß wurde fest verschlossen und die Reaktionsmischung bei 290 W für 10 Minuten unter Rühren und Luftkühlung so bestrahlt, dass die maximale Temperatur ca. 160°C betrug. Durch Integration mittels ¹H-NMR-Spektroskopie konnte ein Umsetzungsgrad von > 98 % ermittelt werden.

### Beispiel 4

1 g eines Glycerin-basierenden Polyalkylenglykoltriamins (Ethylenglykol-Propylenglykol-Mischung) mit einer mittleren Molmasse von 2500 g/mol und 0,6 g Methacrylsäure wurde in ein Druckgefäß mit Rührstab gefüllt. Das Druckgefäß wurde fest verschlossen und die Reaktionsmischung bei 290 W für 10 Minuten unter Rühren und Luftkühlung so bestrahlt, dass die maximale Temperatur ca. 180°C betrug. Durch Integration mittels ¹H-NMR-Spektroskopie konnte ein Umsetzungsgrad von > 98 % ermittelt werden.

### Beispiel 5

20 g eines Methylpolyalkylenglykolamins (Ethylenglykol- Propylenglykol-Mischung) mit einer mittleren Molmasse von 1000 g/mol und 10,4 g Methacrylsäure wurden in ein Druckgefäß mit Rührstab gefüllt. Das Druckgefäß wurde fest verschlossen und die Reaktionsmischung bei 300 W 40 Minuten unter Rühren und Luftkühlung so bestrahlt, dass die maximale Temperatur 200°C nicht überstieg. Zur Aufreinigung wurde die Reaktionslösung nach beendeter Reaktion in 50 ml Dichlormethan aufgenommen. Es wurde einmal mit 50 ml einer 1 normalen HCl-Lösung ausgeschüttelt. Die organische Phase wurde zweimal mit 50 ml einer kaltgesättigter NaHCO₃-Lösung und anschließend mit Wasser gewaschen. Es wurde über Magnesiumsulfat getrocknet und das Lösemittel am Rotationsverdampfer entfernt. Das Produkt wurde mittels Infrarot (IR)- und Kernresonanz (NMR)-Spektroskopie, sowie mittels Massenspektrometrie (MALDI-TOF) charakterisiert. Die Ausbeute betrug 85%.

### Beispiel 6

20 g eines Methylpolyalkylenglykolamins (Ethylenglykol- Propylenglykol-Mischung) mit einer mittleren Molmasse von 2000 g/mol, 8 mg Phenothiazin, 8 mg para-Methoxyphenol und 4,3 g Methacrylsäure wurden in ein Druckgefäß aus Polytetrafluorethylen mit Rührstab gefüllt. Das Druckgefäß wurde fest verschlossen und die Reaktionsmischung unter Rühren für 6 Stunden bestrahlt, wobei die Strahlungsleistung so reguliert wurde, dass die Innentemperatur bei 200°C lag. Durch Integration mittels ¹H-NMR-Spektroskopie konnte ein Umsetzungsgrad von > 90 % ermittelt werden.

### Beispiel 7

25 g eines Polyalkylenglykoldiamins (Ethylenglykol- Propylenglykol-Mischung) mit einer mittleren Molmasse von 1500 g/mol, 7 mg Phenothiazin, 7 mg para-Methoxyphenol und 11,5 g Methacrylsäure wurden in ein Druckgefäß aus Polytetrafluorethylen mit Rührstab gefüllt. Das Druckgefäß wurde fest verschlossen und die Reaktionsmischung unter Rühren für 6 Stunden bestrahlt, wobei die Strahlungsleistung so reguliert wurde, dass die Innentemperatur bei 200°C lag. Durch Integration mittels ¹H-NMR-Spektroskopie konnte ein Umsetzungsgrad von > 92 % ermittelt werden.

### Beispiel 8

30 g eines Resorcin-basierenden Polyalkylenglykoldiamins (Ethylenglykol-Propylenglykol-Mischung) mit einer mittleren Molmasse von 2300 g/mol, 10 mg Phenothiazin, 10 mg para-Methoxyphenol und 11,2 g Methacrylsäure wurden in ein Druckgefäß aus Polytetrafluorethylen mit Rührstab gefüllt. Das Druckgefäß wurde fest verschlossen und die Reaktionsmischung unter Rühren für 6 Stunden bestrahlt, wobei die Strahlungsleistung so reguliert wurde, dass die Innentemperatur bei 200°C lag.
Durch Integration mittels ¹H-NMR-Spektroskopie konnte ein Umsetzungsgrad von > 90 % ermittelt werden.

### Vergleichsbeispiel 1 (thermisches Erhitzen)

20 g eines Methylpolyalkylenglykolamins (Ethylenglykol- Propylenglykol-Mischung) mit einer mittleren Molmasse von 2000 g/mol, 8 mg Phenothiazin, 8 mg para-Methoxyphenol und 4,3 g Methacrylsäure wurden in ein Druckgefäß aus Glas mit Rührstab gefüllt. Das Druckgefäß wurde fest verschlossen und die Reaktionsmischung unter Rühren auf 200°C erwärmt. Bereits vor dem Erreichen der gewünschten Reaktionstemperatur setzte die unerwünschte Polymerisation des Reaktionsgemisches ein.

## Patentansprüche

1. Verfahren zur Herstellung von Amiden der (Meth)acrylsäure, indem man ein Polyetheramin der Formel 1 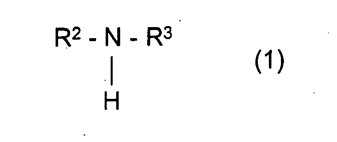 worin R² einen organischen Rest darstellt, der zwischen 2 und 600 Alkoxygruppen umfasst, und R³ Wasserstoff oder einen organischen Rest mit 1 bis 400 Kohlenstoffatomen darstellt, mit (Meth)Acrylsäure mischt, und das Gemisch mit Mikrowellen bestrahlt.

2. Verfahren nach Anspruch 1, worin R³ Wasserstoff, ein Alkylrest mit 1 bis 50 Kohlenstoffatomen, ein Alkenylrest mit 2 bis 50 Kohlenstoffatomen, ein Arylrest mit 6 bis 50 Kohlenstoffatomen, oder ein Alkylarylrest mit 7 bis 50 Kohlenstoffatomen ist.

3. Verfahren nach Anspruch 1 und/oder 2, worin R³ der gleichen Definition wie R² entspricht.

4. Verfahren nach einem oder mehreren der Ansprüche 1 bis 3, worin R³ Aminogruppen enthält.

5. Verfahren nach einem oder mehreren der Ansprüche 1 bis 4, worin R³ der Formel 3 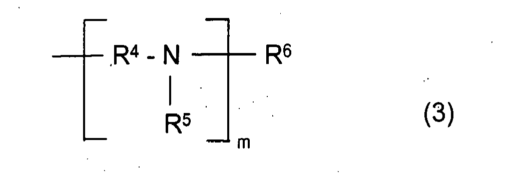 entspricht, worin R⁴ eine zweiwertige Kohlenwasserstoffgruppe mit 1 bis 50 Kohlenstoffatomen und R⁵ und R⁶ jeweils Wasserstoff oder eine einwertige Kohlenwasserstoffgruppe mit 1 bis 50 Kohlenstoffatomen sein können, wobei jedes von R⁴, R⁵ und R⁶ 1 bis 200 Alkoxygruppen umfassen kann, und m für eine Zahl von 1 bis 10 steht.

6. Verfahren nach einem oder mehreren der Ansprüche 1 bis 5, worin das Amin der Formel (1) für ein Polyamin der Formel 8
R⁷(NHR⁸)ₙ (8)
steht, und worin R⁷ für einen n-wertigen organischen Rest mit 2 bis 400 Kohlenstoffatomen, R⁸ für einen Rest wie R³, und n für eine ganze Zahl von 2 bis 20 steht.

7. Verfahren nach einem oder mehreren der Ansprüche 1 bis 6, worin das molare Verhältnis Amin : (Meth)Acrylsäure im Bereich 1: 0,2 bis 15 liegt.

8. Verfahren nach einem oder mehreren der Ansprüche 1 bis 7, worin die Amidierung bei Temperaturen von 40 bis 250°C durchgeführt wird.
